# EUROPEAN PATENT SPECIFICATION

(11) **EP 2 648 712 B1**
(45) Date of publication and mention of the grant of the patent: **21.10.2015**
(21) Application number: 11794722.6
(22) Date of filing: 09.12.2011
(51) Int. Cl.: A61K 31/18, A61K 31/4164, A61P 27/00, A61K 45/06

(54) **ARYLSULFONAMIDE DERIVATIVES FOR THE PREVENTION OR TREATMENT OF MACULAR OEDEMA**
ARYLSULFONAMID-DERIVATE ZUR VORBEUGUNG ODER BEHANDLUNG VON MAKULAÖDEM
DÉRIVÉS D'ARYLSULFONAMIDE POUR PRÉVENIR OU TRAITER LES OEDÈMES MACULAIRES

(30) Priority: 09.12.2010 EP 10306381
(43) Date of publication of application: 16.10.2013
(73) Proprietor: Fovea Pharmaceuticals, 75012 Paris (FR)
(72) Inventor: BELICHARD, Pierre, F-75012 Paris (FR); PRUNEAU, Didier, F-75012 Paris (FR)
(74) Representative: Domenego, Bertrand
(86) International application number: PCT/EP2011/072306
(87) International publication number: WO 2012/076685

(56) References cited:
- WO-A1-2008/080110
- WO-A1-2009/112878
- FR-A1- 2 840 897
- PORRECA F ET AL: "Antinociceptive pharmacology of N-[[4-(4,5-dihydro-1H-imidazol-2-yl) phenyl]methyl]-2-[2-[[(4-methoxy-2,6-dimet hylphenyl)sulfonyl]methylam ino]ethoxy]-N-methylacetamide, fumarate (LF22-0542), a novel nonpeptidic bradykinin B-1 receptor antagonist", JOURNAL OF PHARMACOLOGY AND EXPERIMENTAL THERAPEUTICS, vol. 318, no. 1, July 2006 (2006-07), pages 195-205 URL, XP002627226, ISSN: 0022-3565
- PRUNEAU DIDIER ET AL: "Targeting the kallikrein-kinin system as a new therapeutic approach to diabetic retinopathy", CURRENT OPINION IN INVESTIGATIONAL DRUGS, THOMSON REUTERS (SCIENTIFIC) LTD, LONDON, UK, vol. 11, no. 5, 1 May 2010 (2010-05-01), pages 507-514, XP009145637, ISSN: 2040-3429
- Pruneau D. et al.: "Topical Treatment With the Kinin B1 Receptor Antagonist, FOV2304, Inhibits Diabetic Retinopathy (DR) in Rats", , 2 May 2010 (2010-05-02), XP002627227, ARVO 2010 Abstract Search & Itinerary Builder Retrieved from the Internet: URL:http://www.abstractsonline.complan/Vie wAbstract.aspx?mID=2511&sKey=57cccd01-abc2 -490b-b653-d9691b1088b4&cKey=1e53ce3c-d9ff -4772-8943-dee7a595ffe4&mKey={1EA90E66-C54 8-49E0-9F05-30DA7938D511} [retrieved on 2011-03-08]
- Sanofi Aventis: "Sanofi-aventis to acquire FOVEA Pharmaceuticals, a french biopharmaceutical ophthalmology company", , 1 October 2010 (2010-10-01), pages 1/2-2/2, XP002627228, Press release Sanofi-aventis Retrieved from the Internet: URL:http://www.abingworth.com/_news/Sanofi -aventisbuysFovea.pdf [retrieved on 2011-03-08]
- DATABASE WPI Week 201054 Thomson Scientific, London, GB; AN 2010-K29705 XP002628007, & RU 2 393 824 C1 (UNIV BASHKORTOSTAN MED UFA EYE RES INST) 10 July 2010 (2010-07-10)

## Description

Arylsufonamides are known for example from WO 03/106428, where compounds of formula (I) 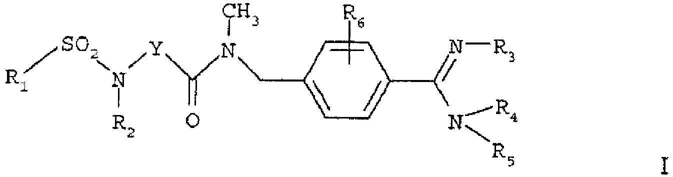 as well as their salts are disclosed, in which:
R₁ represents an aromatic ring that is non-substituted or substituted by one or more atoms or groups of atoms chosen from among the halogens, C₁-C₃ alkyl groups, C₁-C₃ alcoxy groups, nitro, cyano, trifluoromethyl or trifluoromethoxy groups,
R₂ represents a hydrogen atom, or a straight, branched or cyclic hydrocarbon chain having 1 to 4 carbon atoms optionally substituted by a phenyl group, by a CONH₂ group or by one or more fluorine atoms,
R₃ represents a hydrogen atom, a hydroxy group, or with R₄ forms a -CH=N- group or a straight or branched C₂-C₄ alkylene group,
R₄ represents a hydrogen atom or with R₃ forms a -CH=N- group or a straight or branched C₂-C₄ alkylene group,
R₅ represents a hydrogen atom or a C₁-C₃ alkyl group,
R₆ represents a hydrogen atom or a halogen,
Y represents a C₂-C₄ alkylene group, saturated or unsaturated, straight or branched, optionally interrupted between two carbon atoms by an oxygen atom.
By aromatic system is meant a phenyl system, a 1- or 2-naphthyl system or a 2- or 3-thienyl system.

These compounds are described to be useful in the treatment of various forms of pain such as inflammatory hyperalgesia, allodynia, neuropathic pain associated for example with diabetes, neuropathy (constriction of sciatic nerve, lumbago), any form of trauma, surgery (tooth extraction, tonsil removal), interstitial cystitis, inflammatory bowel disease and cancer.

They are also described to be useful in the treatment of any pathology associated with neutrophil migration, such as acute respiratory distress syndrome, psoriasis, chronic lung obstruction, inflammatory bowel diseases, and rheumatoid arthritis.

The compounds, on account of their mode of action, also find use in the treatment or prevention of any pathological condition in which the B1 receptors of bradykinin are involved and in particular are over-expressed.

In addition to the various forms of pain and inflammatory diseases already cited, the compounds of formula (I) may, according to the disclosure of WO03/106428, be used to treat certain respiratory problems such as asthma, bronchitis, pleurisy or rhinitis of allergic and viral origin, certain forms of diabetes, certain skin diseases such as dermatitis, eczema, psoriasis, eye diseases such as glaucoma and retinitis, Alzheimer's disease, septic shock, trauma, especially involving the skull, some cancers, in particular by slowing or inhibiting the proliferation of cancer cells and more particularly cancer of the prostate.

Pruneau D. et al. in "Targeting the kallikrein-kinin system as a new therapeutic approach to diabetic retinopathy", CURRENT OPINION IN INVESTIGATIONAL DRUGS, THOMSON REUTERS (SCIENTIFIC) LTD, LONDON, UK, vol. 11, no. 5, 1 May 2010 (2010-05-01), pages 507-514, and in "Topical Treatment With the Kinin B1 Receptor Antagonist, FOV2304, Inhibits Diabetic Retinopathy (DR) in Rats", 2 May 2010 (2010-05-02), suggest that targeting the pro-inflammatory response via B₁R of the KKS system in diabetic retinopathy is a promising approach for treating retinopathy less invasively. These publications do not even suggest the structure of the compound involved in these studies. It has been found according to the present invention that not all bradykinin B1 receptor antagonists are useful in the prevention, treatment and/ or reduction of macular oedema. It has now been found that a compound of formula (I) as well as its pharmaceutically acceptable salts is useful in the prevention, treatment and/ or reduction of macular oedema, and in particular in the treatment of macular oedema caused by or associated with diabetic retinopathy.

A compound of formula (I) as defined in claim 1 is considered as being particularly useful in the prevention, treatment and/ or reduction of macular oedema, in particular macular oedema associated with or caused by diabetic retinopathy. Such compound is compound N[[5-(4,5-dihydro-1Himidazol-2yl)phenyl]methyl]-2-[2-[-(4-methoxy-2,6-dimethylphenyl)sulfonyl]methylamino]ethoxy]-N-methyl-acetamide or its salts, such as its phosphate, sulphate, or hemisulfate salts.

This compound is exemplified as compound n° 49 of WO 03/106428 and its structural formula is:

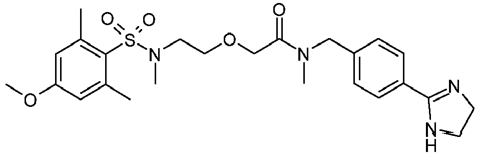

It is referred to in the present specification as "compound n° 49".

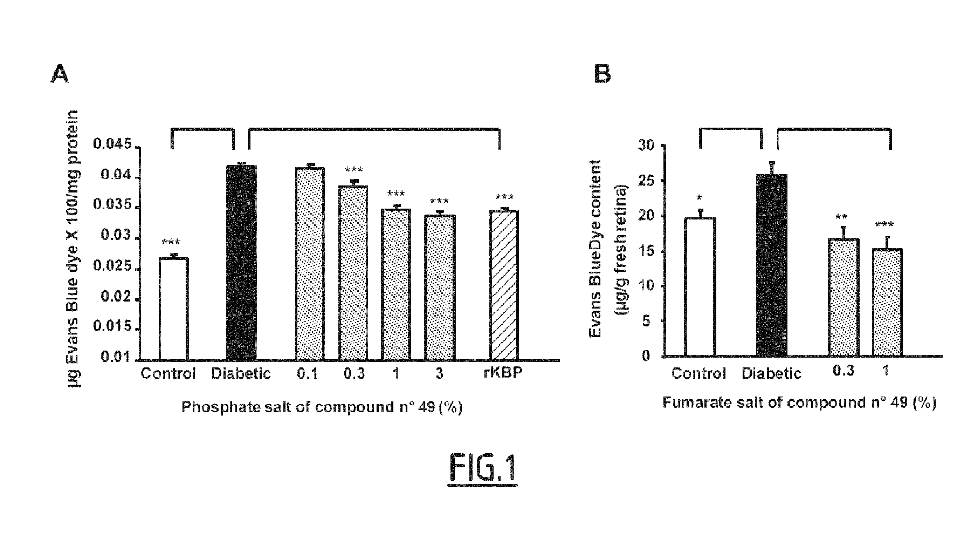

This will be further exemplified by Fig. 1, summarizing the effect of the arylsulfonamide compound n° 49 given as eye drop on retinal vascular permeability in streptozotocin-induced diabetic Brown-Norway (A) and Wistar rats (B).

Diabetic retinopathy is a major complication that affects between 18% and 45% of diabetic patients, a population which is continuously expending worldwide. The number of Americans 40 years or older with diabetic retinopathy and vision-threatening diabetic retinopathy will triple in 2050, from 5.5 million in 2005 to 16.0 million diabetic retinopathy and from 1.2 million in 2005 to 3.4 million for vision-threatening diabetic retinopathy. Increase among those 65 years or older will be more pronounced (2.5 million to 9.9 million for diabetic retinopathy and 0.5 million to 1.9 million for vision-threatening diabetic retinopathy. Moreover incidence of diabetic macular edema over a 10-year period ranges from 20 to 40% among patients diagnosed before and after the age of 30. In this respect, this is the leading cause of blindness in working-age population in the United States. Incidence of diabetic retinopathy varies according to the race, type of diabetes, age and arterial blood pressure status. The prevalence of proliferative retinopathy, macular oedema and vision-threatening retinopathy is in the range of 2-5%, 5-7% and 6-8%, respectively. At early stages, diabetic retinopathy is characterized by ischemic areas of acellular capillaries, and as diabetes progresses over the time, retinal vascular leakage, vascular sprouting, angiogenesis and hemorrhage occur ultimately leading to loss of vision. In diabetic patients, there is a relationship between vision loss and clinically significant macular oedema, defined as vascular plasma leakage leading to fluid accumulation and the deposition of hard exudates within the center of the macula.

As will be further understood from the specification, a compound of formula (I) has been shown to be particularly efficient to treat macular oedema.

In the context of the present specification, the term "macular oedema" has to be understood independently of the underlying disease causing it, and as being associated with any form of retinopathy. It for example includes macular oedema associated with or caused by diabetic retinopathy, age related macular degeneration, retinitis pigmentosa, ocular surgery, retinal vein occlusion (either central vein occlusion or branch vein occlusion, or both). Other examples of macular oedema are associated with or caused by vision threatening retinopathy, proliferative retinopathy, clinically significant macular oedema, or chronic macular edema during diabetic retinopathy, as well as any other stage of what is usually understood as being a diabetic retinopathy.

A compound of formula (I), useful to treat or prevent the specific ophthalmologic diseases of the invention, can also be used in association with other active components. For example, a compound of formula (I) can be used in association with anti VEGF compounds.

In the context of the present specification, by "in association with" it should be understood a co-administration, or a combination of two active principles. The co-administration can be simultaneous, almost simultaneous, or delayed in time by a few days or weeks, for example by up to 4 or 5 weeks.

Vascular endothelial growth factor (VEGF) is an endogenous molecule involved in a number of physiological processes, including blood vessel growth at the foetal stage, during injury healing, or for the growth of new vessels in tissues that have a deficient blood supply. VEGF is also involved in pathological processes, like the development of tumour blood vasculature which allows for growth and spread of the tumour, or the formation of new blood vessels in the eye that eventually contributes to vision loss. Anti-VEGF therapies therefore aim to prevent this abnormal blood vessel formation by blocking VEGF action.

Examples of anti-VEGF compounds include Macugen® (Pegaptanib sodium), Lucentis (ranibizumab), Avastatin® (bevacizumab) RhuFab, or VEGF Trap Eye.

Therefore, in another aspect, a compound of formula (I) is used in association with anti-VEGF compounds for the prevention, treatment and/ or reduction of macular oedema, and in particular of macular oedema associated with or caused by diabetic retinopathy.

A compound of formula (I) can also be used in association with corticosteroids. Corticosteroids are a class of steroid hormones that are produced in the adrenal cortex. Corticosteroids are involved in a wide range of physiologic systems such as stress response, immune response and regulation of inflammation, carbohydrate metabolism, protein catabolism, blood electrolyte levels, and behavior.

Glucocorticoids such as cortisol control carbohydrate, fat and protein metabolism and are anti-inflammatory by preventing phospholipid release, decreasing eosinophil action and a number of other mechanisms. Mineralocorticoids such as aldosterone control electrolyte and water levels, mainly by promoting sodium retention in the kidney.

Corticosteroids are generally grouped into four classes, based on chemical structure. Group A (short to medium acting glucocorticoids) comprises for example hydrocortisone, hydrocortisone acetate, cortisone acetate, tixocortol pivalate, prednisolone, methylprednisolone, and prednisone. Group B comprises triamcinolone acetonide, triamcinolone alcohol, mometasone, amcinonide, budesonide, desonide, fluocinonide, fluocinolone acetonide, and halcinonide. Group C comprises betamethasone, betamethasone sodium phosphate, dexamethasone, dexamethasone sodium phosphate, and fluocortolone. The last group, group D, comprises hydrocortisone-17-butyrate, hydrocortisone-17-valerate, aclometasone dipropionate, betamethasone valerate, betamethasone dipropionate, prednicarbate, clobetasone-17-butyrate, clobetasol-17-propionate, fluocortolone caproate, fluocortolone pivalate, and fluprednidene acetate.

Therefore, in another aspect, a compound of formula (I) is used in association with corticosteroids for the prevention, treatment and/ or reduction of macular oedema, and in particular macular oedema associated with or caused by diabetic retinopathy.

The compound of the present invention is compound n°49 as defined above, as well as its pharmaceutically acceptable salts. This compound can be made according to the processes described in WO 03/106428.

The pharmaceutically acceptable salts of the compound of interest mentioned above under formula (I) may be chosen from sulfate, hemi-sulfate, fumarate, maleate, tartrate, citrate, lactate, succinate, benzoate, camsylate, acetate, phosphate, chloride, bromide, aspartate or pamoate, for example.

### Example 1: Effect of an arylsulfonamide compound of formula (I) on macular oedema

The effect of an arylsulfonamide compound of formula (I) on macular oedema, and in particular on macular oedema associated with or caused by diabetic retinopathy diabetic retinopathy has been investigated as explained below.

Administration of streptozotocin to rodents produces a massive destruction of pancreatic β-cells, and thus, triggers marked elevation of glycemia and development of a diabetic state which, in part, mimics early stages of type 1 diabetes. In this regard, streptozotocin-treated rats develop an inflammatory retinopathy featured by rupture of the blood-retinal barrier, increase of inflammatory mediators, cytokines and growth factors (VEGF, basic fibroblast growth factor), microglial cell activation and leukostasis.

Compound n° 49 (fumarate and phosphate salts) was given as eye drop for 7 days to streptozotocin-diabetic pigmented Brown-Norway and non-pigmented Wistar rats and its effect on retinal edema was then determined.

Male Brown-Norway or Wistar rats were made diabetic by subcutaneous injection of 65 mg/kg intraperitoneal streptozotocin. Rats with a glycemia < 350 mg/dl were discarded from the study. Seven days later, Brown-Norway rats were treated twice daily for 7 days with a single eye drop (10 µl) containing 0.1, 0.3, 1 and 3% phosphate salt of compound n° 49 or its vehicle (Saline Solution). Wistar rats were treated over the same period (from day 7 for 7 days) with a single eye drop (10 µl) containing 0.3 and 1% fumarate salt of compound n° 49 or its vehicle (Saline Solution). On day 15, retinal vascular leakage was determined by measurement of retinal content of Evans Blue dye.

As shown in Fig. 1 retinal vascular permeability was significantly increased in diabetic Brown-Norway and Wistar rats compared to control normoglycemic rats. In diabetic Brown-Norway and Wistar rats, compound n° 49 did not affect glycemia. In diabetic Brown-Norway rats, the phosphate salt of compound n° 49 reduced in dose-dependent manner retinal oedema with a maximum of 55%. In two separate preliminary studies, 1% compound n° 49 eye drops reduced retinal edema with a maximum of 58% and 63% (data not shown). In diabetic Wistar rats, both 0.3 and 1% compound n° 49 fumarate salt abolished retinal vascular permeability (Figure 1). These data show that in two different rat strains made diabetic with streptozotocin, compound n° 49 markedly reduced rupture of the blood retinal barrier.

Fig. 1 shows the effect of compound n° 49 given as eye drop on retinal vascular permeability in streptozotocin-induced diabetic Brown-Norway (A) and Wistar rats (B). More specifically, Fig. 1A is the dose-response of compound n° 49 instilled twice a day for 7 days as eye drops (0.1 to 3%) on retinal vascular leakage in streptozotocin-diabetic Brown-Norway rats. Recombinant tissue kallikrein binding protein (rKBP) was used as a reference drug and was given intravitreally 48 hours before vascular leakage measurement. Values are means ± standard error mean of 12 eyes/group (6 rats). *** means that P<0.001in a Student's t-test.
Fig. 1B shows the effect of compound n° 49 instilled twice a day for 7 days as eye drops (0.3 and 1%) on retinal vascular leakage in streptozotocin-diabetic Wistar rats. Values are means ± standard error mean of 6 to 11 eyes/group. **means P<0.01; *** means P<0.001 ina one-way ANOVA followed by a Student's t-test.

The route by which the phosphate salt of compound n° 49 reaches the retina following topical instillation was investigated to determine whether it is mainly by systemic circulation or by the trans- and/or peri-ocular pathway. Diabetic Brown-Norway rats were treated with 3% compound n° 49 phosphate in a single eye for 7 days or subcutaneously with matching daily dosage (0.6 mg/rat). In eyes treated with compound n° 49, retinal vascular permeability was significantly reduced by 37% (P<0.001) whilst the contralateral eye remained unaffected. In addition, a daily subcutaneous administration of 0.6 mg compound n° 49 phosphate rat had no effect on retinal vascular permeability. These data indicate that compound n° 49 phosphate upon topical administration likely reached retinal vasculature through trans-corneal and scleral/choroidal circulation with no or minimal contribution of systemic redistribution.

In order to explore molecular pathways involved into reduction of retinal vascular permeability by compound n°49 phosphate in diabetic rats, expression of mRNA of cytokines, vasoactive mediators and growth factors was quantified in retina of control and diabetic Wistar rats treated or not with 1% compound n°49 phosphate eye drops for 7 days. Expression of mRNA of B₁R, B₂R, i-NOS, e-NOS, COX-2, ICAM-1, VEGF-R2, VEGF-a, IL-1β and HIF-1α was increased in diabetic rat retina compared to control by 7.5-fold (P<0.05), 5.5, 15- (P<0.01), 6.5- , 8- (P<0.05), 8- , 5- (P<0.05), 12-, 6.5- (P<0.01) and 7-fold (P<0.05), respectively. Expression of TNF-α mRNA remained unchanged. After 7 days treatment with 1% Compound n°49 eye drops, mRNA expression of B₁R, B₂R, i-NOS, e-NOS, COX-2, ICAM-1, VEGF-A, IL-1α and HIF-1α was down-regulated to control level whilst expression of VEGF-A was reduced by 50 %. In accordance with the literature (Gardner and Antonetti, 2008), these data show that the retina of diabetic rats displays inflammatory features. Interestingly, compound n° 49 phosphate blunted the retinal inflammatory response associated with the development of diabetes without affecting glycemia.

The effect of compound n°49 phosphate on leukocyte adhesion to the retinal vasculature of streptozotocin-diabetic Wistar rats was also investigated using the same protocol of administration of compound n°49 phosphate (1% eye drop twice-a-day for 7 days). The number of leukocytes in retinal vessels of diabetic rats was significantly increased compared to control non-diabetic animals (P<0.05). Following treatment with compound n° 49 phosphate, leukostasis in diabetic rats was significantly blunted (P<0.05). These findings are consistent with a reduction of ICAM-1 expression by compound n°49 phosphate, since ICAM-1 has been shown to play a key role in leukocyte adhesion.

Besides, it has been shown that compound n°49 administered under phosphate salt form has an equivalent activity to the fumarate salt.

### Example 2: Comparison of the effect of an arylsulfonamide compound of formula (I) versus another Bradykinin B1 receptor antagonist

### Experimental protocol

In order to induce diabetes, adult Brown-Norway rats (8-12 weeks of age) were given a single intraperitoneal injection of freshly made streptozotocin (STZ) (50 mg/kg of body weight in 10 mmol/L of citrate buffer, pH 4.5). Serum glucose level was examined 2 days after the STZ injection and weekly thereafter. Only the animals with blood glucose levels higher than 350 mg/dLwere used as diabetic rats.

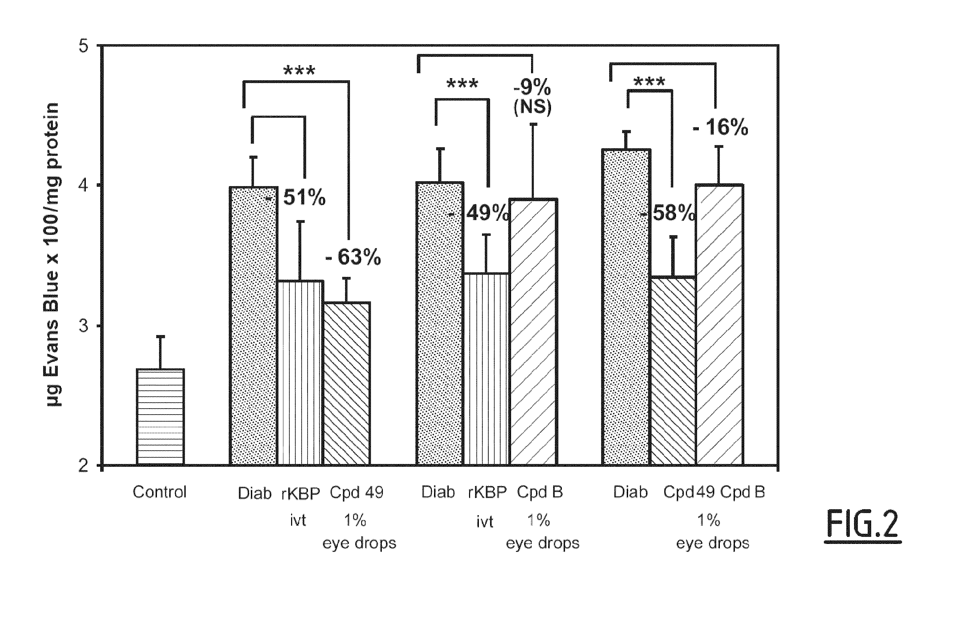

Non-diabetic rats (6-8 rats) were used as positive control group without the treatment with STZ or compound n°49 or B (Control group in Figure 2).

Eight (8) days after the onset of diabetes (i.e. on Day 8), the diabetic rats were separated into groups, with 6-8 rats per group (12-16 eye):

The treatments were as follows:
From Day 8 to Day 14, animals were treated daily with 10 µl ocular instillation in both eyes of 1% of Compound n°49 (solubilised in physiologic serum (0.9% NaCl) as fumarate salt - "Cpd 49" group), 1% of Compound B (solubilised in physiologic serum (0.9% NaCl) as dichlorhydrate salt - "Cpd B" group), or corresponding saline vehicle ("Diab" group, i.e. physiologic serum (0.9% NaCl)). In two experiments, a group of diabetic rats were treated by intravitreal route with recombinant kallikrein-binding protein ("rKBP" group) which was used as a positive reference drug. In such conditions, rKBP has been shown to consistently reduce retinal vascular leakage by approximately 50% (Dr. JX Ma, Charlesson LLC, Oklahoma City, Oklahoma, USA).

Compound B has the following formula:

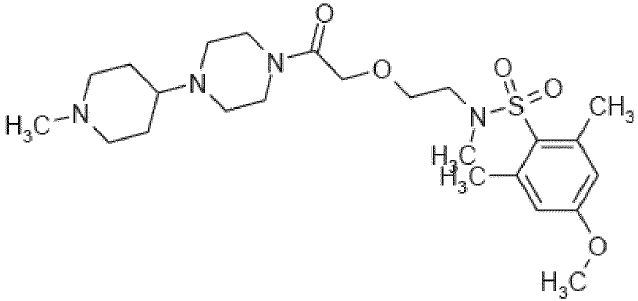

At Day 14, vascular permeability was quantified by measuring Evans Blue-albumin leakage from blood vessels into the retina following a documented protocol (Gao G et al., Diabetologia, 46, 689-698, 2003). Briefly, Evans blue was injected through the femoral vein and circulated for 2 h. Then the rats were infused via the left ventricle with prewarmed PBS. Immediately after perfusion, retina were carefully dissected under an operating microscope and homogenized. Evans blue dye concentration in the retina homogenate was measured using a spectrometer and normalized by total protein concentration.

### Statistical analysis:

Figure 2 represents the results of retinal vascular leakage in STZ diabetic rats (3 studies).

First, vascular leakage mean in rKBP-treated and vehicle-treated rats was compared using a parametric or non parametric test (depending on variance homogeneity). If the difference between the two means was statistically significant, the means of drug- and vehicle-treated groups were compared using a one-way ANOVA (or a non parametric analysis if the variances are not homogeneous) followed by post-hoc testing. In Figure 2 : P values are inferior to 0.001 ("***") according to Dunnet test; "NS" means non-significant.

### Bradykinin B1 Receptor Antagonists:

Both Compound n°49 and Compound B were shown to be Bradykinin B1 Receptor Antagonists. In particular Compound B had an inhibition constant (Ki) of 1.6 nM for the human B1 receptor and had a pA2 of 7.8. Ki was calculated based on the concentration-response curves resulting from competitive binding experiments with [3H]des-Arg10-kallidin, a ligand specific for B1 receptor on HEK293 human cell membranes. pA2 was calculated based on the Schild curve resulting from curves of the concentration-response to des-Arg10-kallidin (B1 receptor agonist) on rat ileum.

### Conclusion:

According to the above results, it has been shown that two Bradykinin B1 Receptor antagonists have not necessary the same activity on macular oedema, especially upon topical administration as demonstrated above. Accordingly, some Bradykinin B1 Receptor Antagonists such as Compound B may have a non-significant activity on macular oedema, whereas a compound of formula (I) (compound n°49) can be used for the prevention, treatment and/ or reduction of macular oedema, in particular associated with or caused by diabetic retinopathy.

## Claims

1. Compound of formula below or one of its pharmaceutically acceptable salts 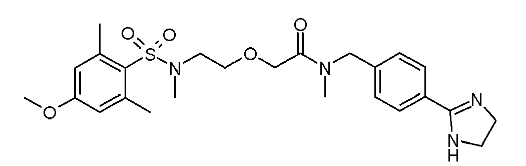 for use in the prevention, treatment and/ or reduction of macular oedema.

2. Compound for use in the prevention, treatment and/ or reduction of macular oedema according to claim 1, **characterised in that** said macular oedema is associated with or caused by diabetic retinopathy.

3. Compound for use in the prevention, treatment and/ or reduction of macular oedema according to any preceding claim wherein the pharmaceutically acceptable salt is phosphate, sulphate, or hemisulfate.

4. Compound for use in the prevention, treatment and/ or reduction of macular oedema according to any preceding claim in association with an anti-VEGF compound.

5. Compound for use in the prevention, treatment and/ or reduction of macular oedema according to claim 4 wherein the anti-VEGF compound is Macugen® (Pegaptanib sodium), Lucentis (ranibizumab), Avastatin® (bevacizumab) RhuFab, or VEGF Trap-eye.

6. Compound for use in the prevention, treatment and/ or reduction of macular oedema according to any of claims 1 to 3 in association with a corticosteroid.

7. Compound for use in the prevention, treatment and/ or reduction of macular oedema according to any preceding claim, **characterized in that** said compound is topically administered.

## Patentansprüche

1. Verbindung der nachstehenden Formel oder eines ihrer pharmazeutisch annehmbaren Salze 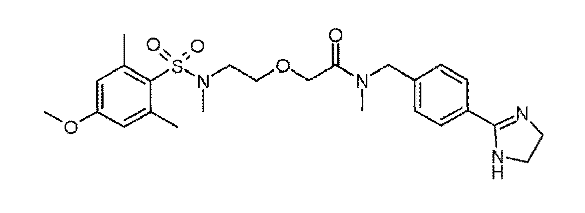 für die Verwendung bei der Prävention, Behandlung und/oder Verringerung von Makulaödem.

2. Verbindung für die Verwendung bei der Prävention, Behandlung und/oder Verringerung von Makulaödem gemäß Anspruch 1, **dadurch gekennzeichnet, dass** das Makulaödem mit diabetischer Retinopathie in Verbindung steht oder davon verursacht ist.

3. Verbindung für die Verwendung bei der Prävention, Behandlung und/oder Verringerung von Makulaödem gemäß einem der vorstehenden Ansprüche, wobei das pharmazeutisch annehmbare Salz Phosphat, Sulfat oder Hemisulfat ist.

4. Verbindung für die Verwendung bei der Prävention, Behandlung und/oder Verringerung von Makulaödem gemäß einem der vorstehenden Ansprüche in Verbindung mit einer anti-VEGF-Verbindung.

5. Verbindung für die Verwendung bei der Prävention, Behandlung und/oder Verringerung von Makulaödem gemäß Anspruch 4, wobei die anti-VEGF-Verbindung Macugen® (Pegaptanib-Natrium), Lucentis (Ranibizumab), Avastatin® (Bevacizumab), RhuFab oder VEGF Trap-eye ist.

6. Verbindung für die Verwendung bei der Prävention, Behandlung und/oder Verringerung von Makulaödem gemäß einem der Ansprüche 1 bis 3 in Verbindung mit einem Corticosteroid.

7. Verbindung für die Verwendung bei der Prävention, Behandlung und/oder Verringerung von Makulaödem gemäß einem der vorstehenden Ansprüche, **dadurch gekennzeichnet, dass** die Verbindung topisch verabreicht wird.

## Revendications

1. Composé de formule ci-dessous ou un de ses sels pharmaceutiquement acceptables 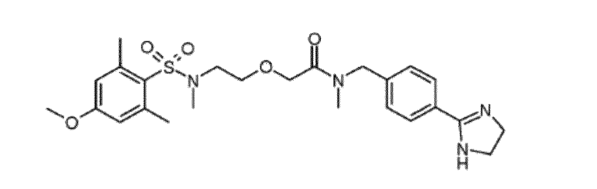 pour utilisation dans la prévention, le traitement et/ou la réduction d'un oedème maculaire.

2. Composé pour utilisation dans la prévention, le traitement et/ou la réduction d'un oedème maculaire selon la revendication 1, **caractérisé en ce que** ledit oedème maculaire est associé à ou causé par une rétinopathie diabétique.

3. Composé pour utilisation dans la prévention, le traitement et/ou la réduction d'un oedème maculaire selon l'une quelconque des revendications précédentes **caractérisé en ce que** le sel pharmaceutiquement acceptable est le phosphate, le sulfate ou l'hémisulfate.

4. Composé pour utilisation dans la prévention, le traitement et/ou la réduction d'un oedème maculaire selon l'une quelconque des revendications précédentes en association avec un composé anti-VEGF.

5. Composé pour utilisation dans la prévention, le traitement et/ou la réduction d'un oedème maculaire selon la revendication 4 **caractérisé en ce que** le composé anti-VEGF est Macugen® (Pegaptanib sodium), Lucentis (ranibizumab), Avastatin® (bévacizumab) RhuFab, ou VEGF Trap-eye.

6. Composé pour utilisation dans la prévention, le traitement et/ou la réduction d'un oedème maculaire selon l'une quelconque des revendications 1 à 3 en association avec un corticostéroïde.

7. Composé pour utilisation dans la prévention, le traitement et/ou la réduction d'un oedème maculaire selon l'une quelconque des revendications précédentes, **caractérisé en ce que** ledit composé est administré par voie topique.
